(19) Europäisches Patentamt
European Patent Office
Office européen des brevets

(11) Publication number: 0 310 194 A1

# (12) EUROPEAN PATENT APPLICATION

(21) Application number: 88202121.5

(22) Date of filing: 29.09.88

(51) Int. Cl.4: C07C 43/13, C07C 43/11, C07C 41/06

(30) Priority: 30.09.87 US 102933

(43) Date of publication of application: 05.04.89 Bulletin 89/14

(84) Designated Contracting States: DE FR GB IT NL SE

(71) Applicant: UNION CARBIDE CORPORATION
39 Old Ridgebury Road
Danbury Connecticut 06817(US)

(72) Inventor: Knopf, Robert John
2657 Lakeview Drive
St. Albans West Virginia 25177(US)

(74) Representative: Smulders, Theodorus A.H.J. et al
Vereenigde Octrooibureaux Nieuwe Parklaan 107
NL-2587 BP 's-Gravenhage(NL)

## (54) Continuous process for producing linear, secondary, aliphatic alcohol ethoxylates.

(57) A continuous process for efficiently producing secondary alcohol ethoxylates of long-chain alpha-olefins which have structural characteristics desirable for specialty surfactant hydrophobe service. This continuous process involves the solvent-less reaction of a long-chain alpha-olefin with ethylene glycol or a lower poly-(oxyethylene)glycol in the presence of a heterogeneous acid catalyst.

PLOT OF DAILY CONVERSION - 1ST CONTINUOUS RUN

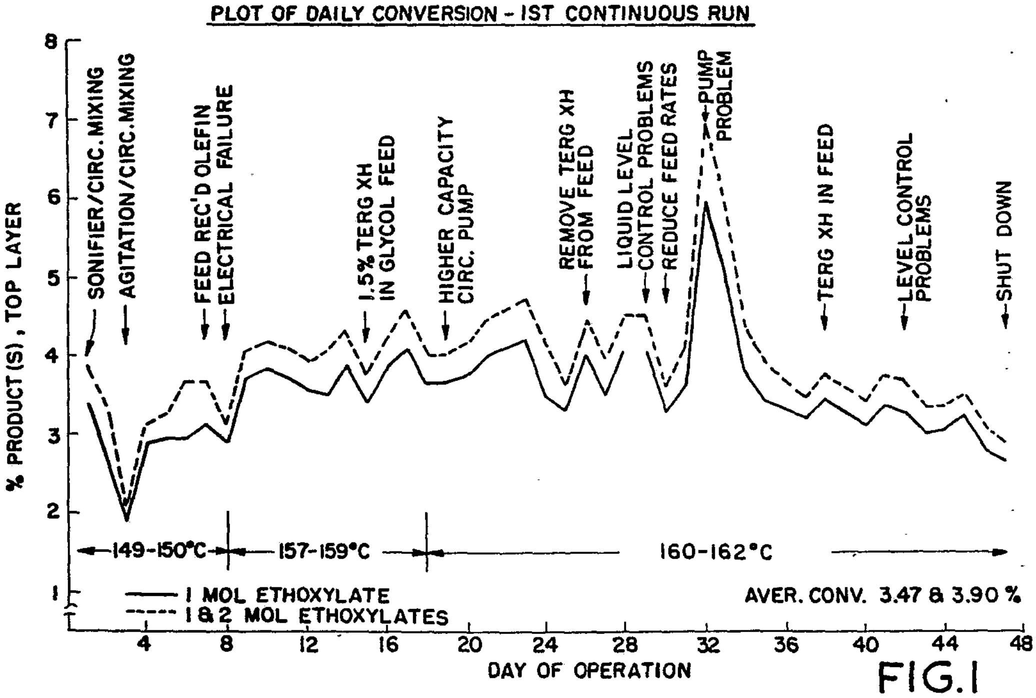


FIG.1

EP 0 310 194 A1

# CONTINUOUS PROCESS FOR PRODUCING LINEAR, SECONDARY, ALIPHATIC ALCOHOL ETHOXYLATES

## Brief Summary of the Invention

### Technical Field

This invention relates to a process for the continuous production of linear, secondary, aliphatic alcohol ethoxylates which have structural characteristics desirable for specialty surfactant hydrophobe service. This continuous process involves the solvent-less reaction of a long-chain alpha-olefin with ethylene glycol or a lower poly(oxyethylene)glycol in the presence of a heterogeneous acid catalyst.

### Background of the Invention

Olefin etherification has long been known in general terms; however, full commercial success in the detergent industry has been elusive. It is known that olefin etherification becomes increasingly less facile with detergent range olefins having a higher molecular weight and a lower degree of substitution around the double bond. Although not investigated widely, attempts have been made to provide secondary alcohol ethoxylates and/or precursors thereof for use in industrial detergent applications.

For example, U.S. Patent 4,139,566 discloses a process of reacting olefins with poly(oxyalkylene)-glycols in the presence of an acid catalyst and a liquid sulfur dioxide solvent to produce the corresponding poly(oxyalkylene)glycol monoalkyl ether. It is stated therein that the use of a sulfur dioxide solvent over a conventional organic solvent provides improved conversion of the olefin and enhanced selectivities to the monoalkyl ether of the poly(oxyalkylene)glycol.

U.S. Patent 4,371,716 describes a process for preparing beta-(sec-alkoxy)ethanol by reacting an olefin with ethylene glycol in the presence of a sulfuric acid or benzenesulfonic acid catalyst and sufficient sulfolane or alkyl-substituted sulfolane solvent to provide a one phase reaction medium, and thereafter extracting the reaction products with an alkane and recovering the beta-(sec-alkoxy)ethanol product from the alkane extract.

Olefin etherification using non-detergent range olefins and simple monofunctional alcohols is exemplified in the art. For example, U.S. Patent 2,067,385 discloses a process for preparing an ether by reacting a secondary-base olefin with an aliphatic alcohol in the presence of a non-basic condensing agent which promotes a condensing action between the olefin and alcohol. Example 1 therein illustrates the production of methyl secondary butyl ether by reacting methyl alcohol with beta-butylene in the presence of sulfuric acid. Example 2 therein illustrates the reaction of ethylene glycol with beta-butylene in the presence of sulfuric acid to produce the mono secondary butyl ether of ethylene glycol.

Copending U.S. Patent Application Serial No. 508,561, filed June 28, 1983, herein incorporated by reference, describes a continuous process for efficiently co-producing secondary alcohols of long-chain olefins and lower carboxylic acid esters by (i) performing an acid/olefin reaction in the presence of a heterogeneous catalyst and (ii) performing an ester-exchange reaction in the presence of an ester-exchange catalyst, e.g., titanium alcoholate. A desirable feature of the process is the ability to produce co-products of choice.

It is therefore an object of the present invention to provide an economically desirable, highly efficient, environmentally clean, solvent-less, continuous process for producing linear, secondary, aliphatic alcohol ethoxylates which have structural characteristics desirable for specialty surfactant hydrophobe service by utilizing the reaction sequence described hereinafter.

## Disclosure of the Invention

This invention relates to a continuous process for producing secondary alcohol ethoxylates from long-chain alpha-olefins comprising:

(a) passing a long-chain alpha-olefin or mixture (optionally in a mixture with internal olefin isomers thereof) together with ethylene glycol or a lower poly(oxyethylene)glycol through a reaction zone in the presence of an acidic heterogeneous catalyst and in the absence of a solvent under conditions at which the corresponding oxyalkylation reaction will occur and thereby producing secondary alcohol ethoxylates and isomers thereof; and

(b) recovering the secondary alcohol ethoxylates and isomers thereof produced in (a).

The process of this invention is uniquely suited to the production of linear, secondary, aliphatic alcohol ethoxylates of the type which are used commercially as hydrophobes in the preparation of high-performance, specialty, nonionic and anionic surfactants.

## Brief Description of the Drawings

Fig. 1 and Fig. 2 each depict the daily weighted average conversions in the form of plots of the secondary alcohol ethoxylate(s) concentration in the top layer of crude reactor effluent versus day of operation for separate continuous runs described more fully hereinafter.

## Detailed Description

As indicated above, this invention relates to a continuous process for producing secondary alcohol ethoxylates from long-chain alpha-olefins comprising:

(a) passing a mixture of a long-chain alpha-olefin, and optionally the internal olefin isomers thereof, together with ethylene glycol or a lower poly(oxyethylene)glycol through a reaction zone in the presence of an acidic heterogeneous catalyst and in the absence of a solvent under conditions at which the corresponding oxyalkylation reaction will occur, thereby producing mixtures of isomeric secondary alcohol ethoxylates; and

(b) recovering the mixture of isomeric secondary alcohol ethoxylates produced in (a).

The process of this invention represents a highly desirable route to detergent range, secondary alcohol ethoxylates. The products obtained from this process have all the structural characteristics desirable for specialty surfactant hydrophobe service, namely, a distribution of carbon numbers within the general range of $C_{10-16}$ C 6 linear chains with all secondary hydroxyl functionality, and a broad isomer distribution. In addition, these products are free of troublesome, e.g., color forming, impurities. The properties and performance characteristics of surfactants derived from hydrophobes made via this process are highly desirable from a commercial standpoint.

The following reaction scheme illustrates the continuous process of this invention and the products which result from its practice:

$$R^1 - CH = CH_2 \quad + \quad HO - [CH_2CH_2O]_x - H$$

$$\xrightarrow{H^+ \text{ Resin}} \quad R^1 - \underset{\substack{|\\ CH_3}}{CH} - O - [CH_2CH_2O]_x - H$$

wherein $R^1$ is a $C_8$-$C_{14}$ radical, preferably a $C_{10}$-$C_{12}$ radical, and x is a value of from 1 to 3. Thus, if $R^1$ is $C_{12}H_{25}$ and x is 1, the above reaction scheme shows the formation of the 1 mol ethoxylate (some 2 mole ethoxylate present) of secondary tetradecanol (mixture of several isomers) as the product.

In the continuous process of this invention, the alpha-olefin and ethylene glycol or lower poly-(oxyethylene)glycol are brought together in a reaction zone at the head end of the process train while the secondary alcohol ethoxylate is recovered at the tail end of the process train. A particularly advantageous feature of the process of this invention is its simplicity. For example, the only operations (other than the reaction itself) which are required between the head and tail ends of the process train are several continuous distillations which serve to remove/separate unreacted raw materials for recycle.

The process of this invention is generally described as follows, using for purposes of illustration the case wherein a mixture of $C_{13}/C_{14}$ alpha-olefins and diethylene glycol are the raw materials and a mixture of secondary $C_{13}/C_{14}$ alcohol ethoxylates (mixed isomers) is the product. The product is primarily the 1 mol and 2 mol ethoxylates of the secondary $C_{13}/C_{14}$ alcohol.

It is optional, but desirable, to insert prior to the oxyalkylation step a pre-reaction "clean-up" reactor to remove tramp metal ions. This may desirably be a bed packed with the oxyalkylation catalyst, e.g., Amberlyst 15, but operated at a relatively low temperature, e.g., about 60° C. This temperature is chosen to be below the reaction temperature of the oxyalkylation reaction; thus, the catalyst acts essentially as an ion exchange resin instead of an oxyalkylation catalyst.

A preheated mixture of diethylene glycol (in excess, preferably) and $C_{13}/C_{14}$ alpha-olefins is passed first through an ion-exchange resin bed and then through one or more heated, fixed-bed type reaction zones containing strongly acidic resin(s) as catalyst(s). No solvent is required for this process. Temperature and pressure in the oxyalkylation step are not considered narrowly critical, but should be chosen to maintain all reactants in the liquid phase. Conversion rate will increase with increasing temperature, but catalyst deterioration will also thereby be accelerated. The homogenous effluent from the reaction zone contains unreacted diethylene glycol and unreacted $C_{13}/C_{14}$ olefins (in partially isomerized form). A typical range for olefin conversion is 20-35%; the actual conversion achieved is dependent upon a host of variables including temperature, residence time in the reactor, glycol: olefin mol ratio, catalyst, catalyst age, extent of isomerization in the recycle olefin feed, etc., but must necessarily be less than 100% because of the reversible nature of the reaction.

The homogeneous effluent from the reactor is pumped directly into the column section of a stripping still (operated at or below atmospheric pressure) wherein diethylene glycol is flashed overhead and recycled to the reaction system while a substantially glycol-free effluent is removed from the base of the still. This stream is pumped into the mid- or upper-column section of a vacuum stripping still wherein partially isomerized $C_{13}/C_{14}$ olefins are recovered overhead for recycle to the reaction system while a substantially olefin-free effluent is removed from the base of the still. When this stripping still is operating in a steady-state mode at the appropriate conditions of temperature, pressure, feed rate and reflux ratio, both the overhead and the bottoms stream will contain less than 2% contaminants.

The $C_{13}/C_{14}$ secondary alcohol ethoxylate mixture is used directly as a hydrophobe in the manufacture of non-ionic surfactants (or as an intermediate in the manufacture of anionic surfactants); these conversions are accomplished by known art procedures however, and this technology does not constitute a part of the present invention.

Olefins suitable for use as raw materials in this continuous process for manufacturing detergent-range secondary alcohol ethoxylates are the linear, terminal species of type (I) below and the linear, internal species of type (II) below.

(I) $R^1$-CH = $CH_2$ (II) $R^2$-CH = CH-$R^3$

The type (I) olefins (so-called alpha-olefins) are the primary species used initially in the process and are known materials prepared by conventional methods; however, the type (I) olefins are partially isomerized into the type (II) olefins by the acidic resin bed in the reaction zone; consequently, the type (I) and type (II) olefins are both present in the recycle process stream. It is this concurrent isomerization reaction which is responsible for the broad isomer distribution present in the product secondary alcohol ethoxylates. For olefins of type (I), $R^1$ is a linear, unsubstituted alkyl radical of from 8-14 carbon atoms (i.e., n-octyl through n-tetradecyl); in the type (II) olefins, $R^2$ and $R^3$ are linear, unsubstituted alkyl radicals whose chain lengths must satisfy the relationship $R^2 + R^3 = C_8$-$C_{14}$. The number of internal isomers possible in olefins of type (II) is 4 with decene and undecene, 5 with dodecene and tridecene, 6 with tetradecene and pentadecene, and 7 with hexadecene.

In the practice of this invention, mixtures of olefins are desirably employed because this approach gives

the best performance characteristics in the surfactant product. For example, mixtures of 12, 13, and 14 carbon species are often ideal in surfactant applications. Due to certain separations which must be performed in this process, mixtures containing more than 2 adjacent carbon number species are best prepared by blending the products from separate runs, each made with mixtures of species two or less carbon numbers wide. Thus, if a $C_{12}$-$C_{15}$ secondary alcohol is desired, the preferred approach is to combine (blend) the products from separate runs made with a $C_{12}/C_{13}$ olefins mixture and a $C_{14}/C_{15}$ olefins mixture.

The stipulation that $R^1$, $R^2$ and $R^3$ in the generic formulae (I) and (II) be linear is made from a final product (surfactant) performance viewpoint only; branched hydrophobe structures are generally less desirable than their linear counterparts because they are more difficultly biodegradable and therefore less "clean" environmentally. In practice, minor amounts of branched structures will be present in the final product because the "linear alpha-olefin" feedstocks derived from petroleum cracking often contain small quantities of branched materials which arise from skeletal rearrangements which occur during the cracking operations. Thus, the stipulation that the olefin feedstocks be linear is not made with the intention of excluding from process suitability linear olefins having minor content of branched structures. Rather, the intention is to emphasize the desirability of avoiding intentional introduction of branched structures into the olefin feedstocks.

Glycols, i.e., ethylene glycol and lower poly(oxyethylene)glycols, suitable for use in the continuous process of this invention are those of the generic structure $HO\text{-}[CH_2CH_2O]_x\text{-}H$ wherein x is a value of from 1 to 3. Illustrative glycols which can be used as reactants in the process of this invention include ethylene glycol, diethylene glycol and triethylene glycol.

The amount of alpha-olefin and ethylene glycol or lower poly(oxyethylene)glycol used as reactants in the process of this invention can vary over a wide range. In general, the molar ratio of the alpha-olefin to the glycol can range from about 0.25:1 to about 1.5:1. Preferably, an equimolar amount or a slight excess of the glycol reactant is employed; in any event, conversions of the reactants are not complete in this reaction.

The catalysts suitable for use in the continuous process of this invention are solid acid catalysts. These resins not only catalyze the glycol reaction with both the alpha-olefins and the internal olefins, but they do so at rates which permit their use in a packed (fixed) bed mode where contact times must necessarily be rather short. Thus, these resins are ideally suited for continuous process service. As heterogeneous catalysts, these resins offer all the other advantages inherent in such materials, e.g., ease of handling and storage, elimination of catalyst removal problems, regeneratability, low unit cost, low corrosivity, and ease of ultimate disposal through land-fill measures, among others. In the present process, the formation exclusively of secondary alcohol ethoxylates (as a mixture of isomers) from either alpha-olefins or internal olefins is desirable because the properties of the surfactants derived from the secondary alcohol ethoxylate products are enhanced by the presence of a multiplicity of isomers.

Two classes of strong acid resins can be used as catalysts in the oxyalkylation step of the instant process, namely, 1) copolymeric, high flourine content aliphatic sulfonic acids of the type exemplified by DuPont's Nafion® H, and 2) macroreticular, cross-linked sulfonated styrene/divinyl benzene copolymers of the type exemplified by Rohm and Hass' Amberlyst® 15, Amberlyst XN-1005, Amberlyst XE-372, etc.

The macroreticular resins are readily available, relatively inexpensive, and easily amenable to structural modification in order to "tailor" the catalyst for optimum performance in a specific reaction system. It should be emphasized that not all macroreticular, strong acid resins will function as effective catalysts in the process of this invention. Physical properties such as resin porosity, surface area and crosslink density are enormously important in determining the suitability of a given resin for use with a given reaction system. Catalyst selection will be a matter of choice, and selection of specific physical properties will depend upon the particular reactants involved. It may be said in general that the physical properties of the catalyst must be such as to permit ready diffusion of the reactant molecules into the catalyst structure to permit adequate contact with the catalytically active surfaces.

Cross-link density is considered to be particularly significant in this regard since it directly affects the degree of swelling of the catalyst resin. In the case of styrene/divinyl benzene copolymers, cross-link density is considered to be directly related to the amount of divinyl benzene present. In general, a cross-link density in the range of about 18-21% will produce good results, while a cross-link density of about 12% or less will be unsatisfactory. Similarly, a pore volume of more than about 30%, preferably about 30-35%, is desirable.

Nafion® H is a particularly preferred catalyst for use in the continuous process of this invention. This catalyst affords high conversion rates irrespective of reaction temperature and also possesses desired chemical and physical stability characteristics under rigorous use conditions.

The amount of catalyst which can be used in the continuous process of this invention is a catalytically

effective amount and can vary over a wide range. Generally, the amount of catalyst employed can range from about 0.01 weight percent to about 10 weight percent or higher based on the total weight of the reactants. Higher catalyst concentrations in general provide increased conversions.

The reaction temperature is not narrowly critical and can be varied over a wide range. The process of this invention is normally conducted at a temperature in the range of from about 125° C to about 185° C, preferably from about 140° C to about 170° C. Higher reaction temperatures in general provide increased conversions, but catalyst deterioriation will also thereby be accelerated.

Reaction pressures are not critical. The process of this invention can be conducted at either subatmospheric, atmospheric or superatmospheric pressure. For convenience, the reaction is usually conducted at atmospheric or autogenous pressure.

The reaction time period is not narrowly critical and can vary over a wide range. The process of this invention is effected over a period of time sufficient to produce the secondary alcohol ethoxylates. Generally, when operating under preferred reaction conditions, reaction times of from about 2 hours or less to about 12 hours are sufficient to complete the reaction of the ' alpha-olefin with ethylene glycol or the lower poly(oxyethylene)glycol. Reaction time is influenced by the degree of mixing. Efficient mixing contributes to accelerate the reaction rate.

Other ingredients can optionally be employed in the process in this invention such as surfactants. Such surfactants are conventional materials known in the art. Suitable surfactants include, for example, conventional nonionic and anionic materials such as poly(oxyalkylene)glycol ethers and esters, sodium oleate, sulfates of higher fatty alcohols, aliphatic or aromatic sulfonates and the like. The amount of surfactant employed in the process of this invention can range from about 0.0001 weight percent or less to about 1.0 weight percent or greater (based on the weight of the entire reaction mass).

The process of this invention is conducted in a continuous fashion. The reaction can be conducted in a single reaction zone or in a plurality of reaction zones, in series or in parallel or it may be conducted continuously in an elongated tubular zone or series of such zones. The materials of construction employed should be inert to the reactants during the reaction and the fabrication of the equipment should be able to withstand the reaction temperatures and pressures. Means to introduce and/or adjust the quantity of reactants or ingredients introduced continuously into the reaction zone during the course of the reaction can be conveniently utilized in the process especially to maintain the desired molar ratio of the reactants.

The process is preferably conducted in either glass lined, stainless steel or similar type reaction equipment. The reaction zone can be fitted with one or more internal and/or external heat exchanger(s) in order to control undue temperature fluctuations, or to prevent any possible "runaway' reaction temperatures. In preferred embodiments of the process, agitation means to vary the degree of mixing the reaction mixtures can be employed. Mixing by vibration, shaking, stirring, rotation, oscillation, ultrasonic vibration or the like are all illustrative of the types of agitation means contemplated. Such means are available and well known to those skilled in he art.

The following experiments are illustrative of the continuous process of this invention.

The laboratory apparatus used for conducting the glycol/olefin reaction in the continuous mode was suitable for unattended round-the-clock operation. Basically, the reactor was of the continuous-stirred tank-design with mixing provided by conventional agitation, circulation, or both. A standard glass resin kettle was modified with a stainless steel top containing the required openings for feed lines, circulating lines, stirrer, temperature probe, reflux condenser, and glass U-tube for liquid level control.

Nafion®H catalyst, as 1/4" x 1/2" pieces cut from a sheet of the membrane form of the polymer, was contained in a stainless steel, lid-covered "basket" which was fabricated so as to fit snugly against the walls of the kettle. Feeds were metered out of graduated vessels into the reactor and crude make was removed from the reactor by means of Hughes model piston pumps; the olefin reactant, i.e., 1-dodecene, was fed as one stream and the ethylene glycol reactant as the other. In cases where TERGITOL®XH emulsifier was used in the system, it was dissolved in the ethylene glycol and fed with that stream. In order to ensure homogeneity of the ethylene glycol feed stream when emulsifier was present in the mix, the ethylene glycol feed tank was equipped with an agitator and a jacket to permit continuous stirring and heating. Liquid level control in the reactor was maintained at the desired 1250 milliliters point by means of high and low level photoelectric eyes which were clamped on the external arm of the glass U-tube liquid level controller. The photo-cells were wired back to the feed and make pumps so that the appropriate pump was shut off/turned on as required to maintain a constant liquid level in the reactor. Heating of the reaction mixture was supplied by an electric mantle of the tall "basket" type designed for use with a resin kettle. Temperature control was maintained by use of a Honeywell controller with all monitored temperatures on the system being read on "trendicator" digital recorders. During unattended operation, the reactor temperature was also recorded continuously on a panel board chart recorder. The product make which was continuously removed

from the reactor through heated jacketed tubing was transferred into a jacketed, graduated glass receiver of sufficient capacity (2 liters) to safely contain the volume of crude product make generated during the overnight, unattended period of operation. The product make was invariably two-phase in nature so that the volume of each phase was easily readable and the individual phases were readily collectible in separate sample bottles. Volatile species reaching the reflux condenser were removed from the system continuously in a Dean-Stark trap.

Two continuous runs, one of 47 days duration and the other of 45 days duration, were conducted in the laboratory continuous-stirred-tank reaction system described above.

Both continuous runs were monitored for conversion by gas chromatography analysis of the individual top and bottom layers of samples collected twice daily. Figures 1 and 2 depict the daily weighted average conversions in the form of plots of the secondary alcohol ethoxylate(s) concentration in the top layer of crude reactor effluent versus day of operation. In these plots the solid line represents the concentrations of the 1-mol ethoxylate product while the dashed line represents the concentrations of the combined 1-mol and 2-mol ethoxylates. The chronology of key parameter changes during the run is also given on the Figures 1 and 2 plots; this is particularly evident in continuous run 1 which featured a large number of parameter changes throughout its course. The analytical data from which the Figures 1 and 2 plots were prepared was obtained, as indicated hereinabove, by gas chromatography scanning of the two individual liquid layers from each sample collected followed by weight averaging of the values for the two samples/day so as to give a single daily average value. The gas chromatography results were expressed in weight percent directly, virtually all of the peaks having been identified so that conversion factors were available. The results of distillation studies conducted subsequent to the conclusion of the continuous runs confirmed the basic accuracy of the gas chromatography analytical program. The only species found and/or considered to be present in the effluent streams which were not picked up in the gas chromatography scans were residues (probably aldol type oils) in the top layer (typically about 0.5-0.7 percent in concentration) and tetraethylene glycol (typically about 0.7-0.9 percent) in the lower layer. Water, which was known to be present in substantial quantities in the bottom layer, was obviously also undetected by FI gas chromatography. In the first continuous run, a considerable portion of the water present was removed in the Dean Stark Trap; this was determined wet-chemically while the water remaining in the bottom layer of the reactor effluent was disregarded. In the second continuous run, the water content of the composited bottom layers from the effluent was determined wet-chemically because very little Dean Stark Trap material was collected during the second run. The gas chromatography results for the second run were then adjusted to reflect the water level in the bottom layer. Since this procedure was not followed during continuous run 1, the values reported for the various glycols and volatile species are probably much less accurate in the case of continuous run 1 than in that of continuous run 2. From a reactor operational viewpoint, the greatly reduced level of Dean Stark Trap volatiles in the second continuous run undoubtedly reflects the fact that the generally lower reaction temperatures of continuous run 2 caused the volatiles boil-up/carry-over to be substantially reduced in magnitude.

The results of the two continuous runs are summarized in Table I hereinbelow. Generally, these results confirm the heterogeneously-catalyzed olefins/glycol addition as a reversible reaction characterized by low conversions (per pass), high efficiencies to 1-mol and 2-mol ethoxylates based upon the olefin reactant, low efficiencies to 1-mol and 2-mol ethoxylates based upon the ethylene glycol reactant, and a general insensitivity to changes in reaction parameters other than catalyst concentration and reaction temperature.

Upon the addition of Tergitol®XH surfactant to the process, Fig. 1 shows that the conversion increased immediately at day 15, decreased immediately upon its removal at day 26, and exceeded the overall average conversion (3.47%) for the entire continuous run on all but 2 days during this period in which the surfactant was present in the feed.

TABLE I

SUMMARY OF 1ST and 2ND CONTINUOUS RUNS

| | 1st Run | 2nd-Run |
|---|---|---|
| Reactor Residence Time, Hours | 8-10 | 8-10 |
| Run Length, Days | 47 | 45 |
| Total Pounds of Reactant Fed | 187.57 | 207.16 |
| Ethylene Glycol Fed, Mols | 360.31 | 447.2 |
| 1-Dodecene Fed, Mols | 372.75 | 393.48 |
| Mol Ratio, Olefin:Glycol | 1.04 | 0.88 |
| Material Balance, % | 98.8 | 98.75 |
| Nafion®H Charge, Grams | 37 | 93 |
| Conversions, % | | |
| Dodecene | 3.81 | 6.94 |
| Ethylene Glycol | 20.4 | 24.83 |
| Efficiencies, %[a] | | |
| On Dodecene | 82.1 | 79.9 |
| On Ethylene Glycol | 16.3 | 19.6 |
| Productivities, %[b] | | |
| Lbs/Lb. Feed | 0.0322 | 0.0534 |
| Lbs/Lb. Catalyst | 73.6 | 52.9 |
| Weight Ratio, 1 Mol Ethyoxylate: | | |
| 2 Mol Ethoxylate: Secondary Alcohol | 16.8:2.2:1.0 | 11.5:1.4:1.0 |
| Catalyst Characteristics | | |
| Turnover No.[c] | 73.6 | 52.9 |
| Lbs Feed/Lb. Catalyst | 2230 | 1021 |
| Final Activity | | |
| By % of Average Conversion | 79.3 | 104.7 |
| By Equiv. Wt. Titration | - | 71.7 |
| Swollen Weight, % of Original | 245 | 248 |

TABLE I (Cont.)

SUMMARY OF 1ST and 2ND CONTINUOUS RUNS

| | 1st Run | 2nd-Run |
|---|---|---|
| Products/By-Products Made, Mols | | |
| 1-Mol Ethoxylate | 9.87 | 18.02 |
| 2-Mol Ethoxylate | 1.07 | 1.86 |
| $C_{12}$ Alcohols (Secondary) | 0.73 | 1.94 |
| Diethylene Glycol | 22.41 | 32.39 |
| Triethylene Glycol | 3.23 | 6.82 |
| Dioxane | 2.16 | 5.70 |
| Methyl Dioxolane | 1.74 | 1.39 |
| Water | 10.29[d] | 52.58[e] |

a To useful products; i.e., secondary alcohols + 1-mole and 2-mol ethoxylates.

b To useful products, i.e., secondary alcohols + 1-mol and 2-mol ethoxylates.

c At time of shutdown; no attempt to determine ultimate value.

d Water present only in distilled volatiles (collected in Dean-Stark Trap).

e Water present in effluent bottom layer, by KF analysis.

Although the invention has been illustrated by the preceding experiments, it is not to be construed as being limited thereby; but rather, the invention encompasses the generic area as hereinbefore disclosed. Various modifications and embodiments can be made without departing from the spirit and scope thereof.

## Claims

1. A continuous process for producing secondary alcohol ethoxylates from long-chain alpha-olefins comprising:

(a) passing a mixture of a long-chain alpha-olefin, and optionally the internal olefin isomers thereof, together with ethylene glycol or a lower poly(oxyethylene)glycol through a reaction zone in the presence of an acidic heterogeneous catalyst and in the absence of a solvent under conditions at which the corresponding oxyalkylation reaction will occur, thereby producing mixtures of isomeric secondary alcohol ethoxylates; and

(b) recovering the mixture of isomeric secondary alcohol ethoxylates produced in (a).

2. The process of claim 1 wherein the alpha-olefin is a linear, unsubstituted olefin of from 10 to 16 carbon atoms.

3. The process of claim 2 wherein the alpha-olefin is a mixture of linear, unsubstituted olefins of from 10 to 16 carbon atoms.

4. The process of claim 3 wherein the alpha-olefin is a mixture of linear, unsubstituted olefins of from 12 to 14 carbon atoms.

5. The process of claim 2 wherein the linear, unsubstituted olefin comprises an alpha-olefin of from 10 to 16 carbon atoms and internal olefin isomers thereof.

6. The process of claim 1 wherein the lower poly(oxyethylene)glycol is selected from diethylene glycol and triethylene glycol.

7. The process of claim 1 wherein the molar ratio of alpha-olefin to ethylene glycol or lower poly-(oxyethylene)glycol is from about 0.25:1 to about 1.5:1.

8. The process of claim 1 wherein the acidic heterogeneous catalyst comprises a copolymeric, high fluorine content aliphatic sulfonic acid, or a macroreticular, cross-linked sulfonated styrene/divinyl benzene copolymer.

9. The process of claim 1 wherein the acidic heterogeneous catalyst is present in an amount of from about 0.01 weight percent to about 10.0 weight percent based on the total weight of the reaction mass.

10. The process of claim 1 wherein the reaction temperature is from about 125° C to about 185° C.

11. The process of claim 1 wherein the reaction period is from about 2 hours to about 12 hours.

12. The process of claim 1 wherein a surfactant is added to the reaction.

13. The process of claim 12 wherein the surfactant is a poly(oxyethylene)glycol monoalkyl ether.

14. The process of claim 12 wherein the surfactant is added in an amount of from about 0.0001 weight percent to about 1.0 weight percent based on the total weight of the reaction mass.

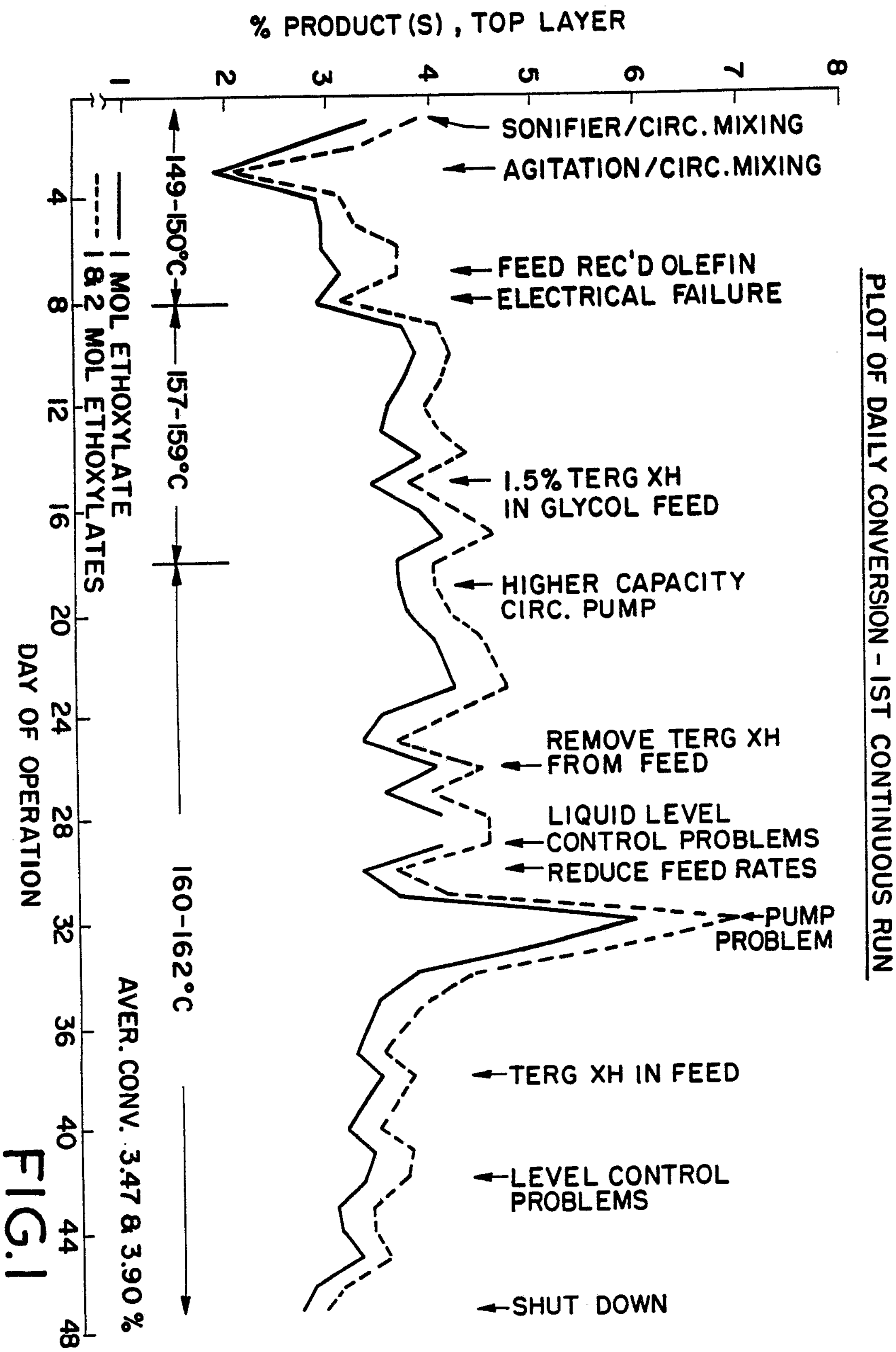
PLOT OF DAILY CONVERSION - 1ST CONTINUOUS RUN
% PRODUCT (S) , TOP LAYER
1
2
3
4
5
6
7
8
DAY OF OPERATION
4
8
12
16
20
24
28
32
36
40
44
48
149-150°C
157-159°C
160-162°C
1 MOL ETHOXYLATE
1 & 2 MOL ETHOXYLATES
AVER. CONV. 3.47 & 3.90 %
SONIFIER/CIRC. MIXING
AGITATION/CIRC. MIXING
FEED REC'D OLEFIN
ELECTRICAL FAILURE
1.5% TERG XH
IN GLYCOL FEED
HIGHER CAPACITY
CIRC. PUMP
REMOVE TERG XH
FROM FEED
LIQUID LEVEL
CONTROL PROBLEMS
REDUCE FEED RATES
PUMP
PROBLEM
TERG XH IN FEED
LEVEL CONTROL
PROBLEMS
SHUT DOWN

FIG. 1

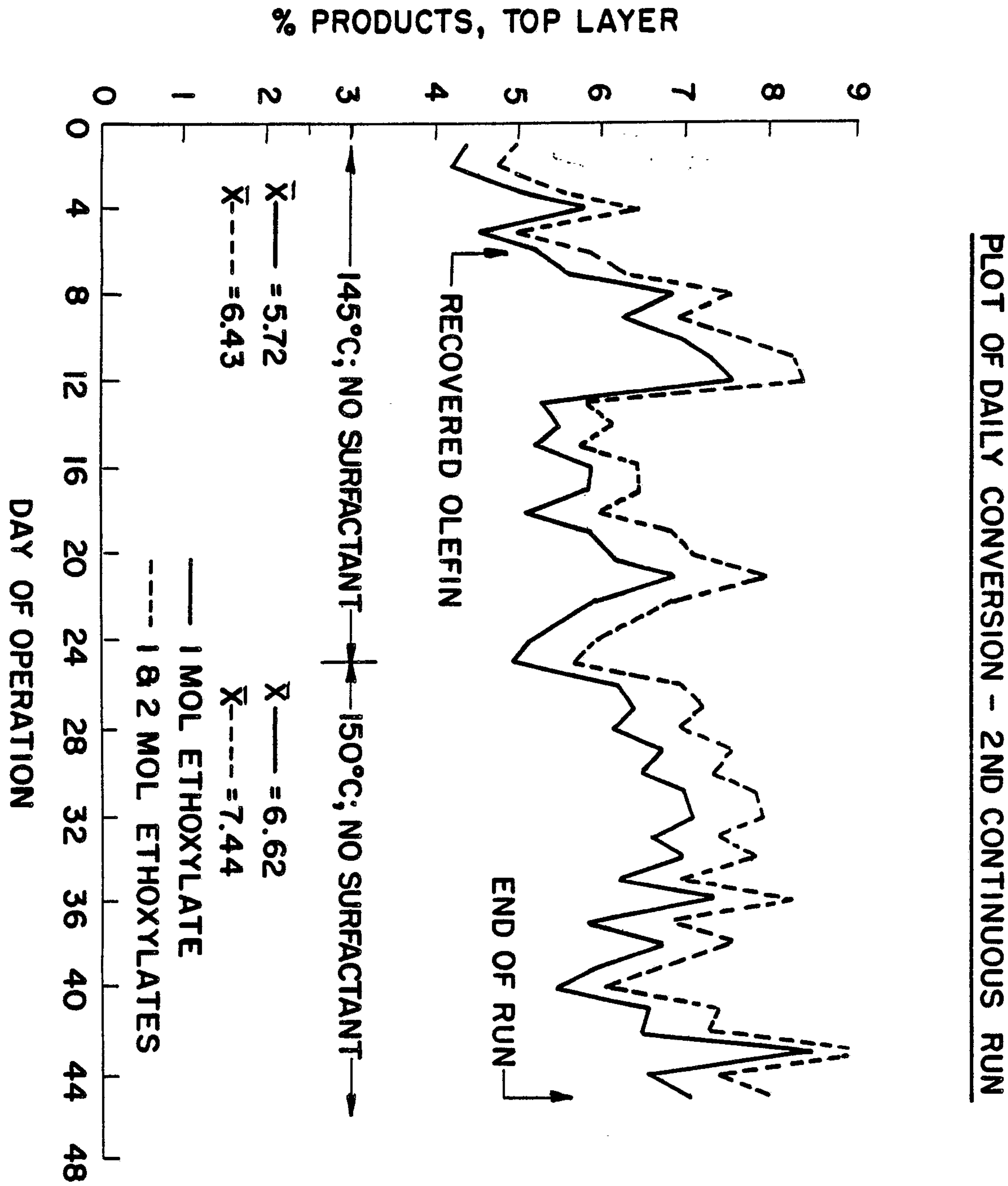
PLOT OF DAILY CONVERSION - 2ND CONTINUOUS RUN
% PRODUCTS, TOP LAYER
0
1
2
3
4
5
6
7
8
9
0
4
8
12
16
20
24
28
32
36
40
44
48
DAY OF OPERATION
RECOVERED OLEFIN
END OF RUN
145°C; NO SURFACTANT
150°C; NO SURFACTANT
X̄—— =5.72
X̄- - - - =6.43
X̄—— =6.62
X̄- - - - =7.44
—— 1 MOL ETHOXYLATE
- - - - 1 & 2 MOL ETHOXYLATES

FIG.2

European Patent Office

# EUROPEAN SEARCH REPORT

Application Number

EP 88 20 2121

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | DE-A-2 450 667 (NIPPON OIL) * Claims; example 3 * --- | 1-14 | C 07 C 43/13<br>C 07 C 43/11<br>C 07 C 41/06 |
| A | EP-A-0 001 651 (SHELL INTERNATIONALE RESEARCH) * Claims; example 1 * --- | 1-14 | |
| D,A | US-A-4 371 716 (T.E.PAXSON) * Abstract * --- | 1-14 | |
| D,A | US-A-2 067 385 (T.W.EVANS) * Examples * --- | 1-14 | |
| A | EP-A-0 129 912 (UNION CARBIDE) * Abstract; page 8; example 1 * ----- | 1-14 | |

TECHNICAL FIELDS SEARCHED (Int. Cl.4)

C 07 C 41/00
C 07 C 43/00

The present search report has been drawn up for all claims

| Place of search | Date of completion of the search | Examiner |
|---|---|---|
| THE HAGUE | 15-12-1988 | WRIGHT M.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)